**Europäisches Patentamt**

**European Patent Office**

**Office européen des brevets**

(19)

(11) **EP 1 148 329 A1**

(12) **EUROPÄISCHE PATENTANMELDUNG**

(43) Veröffentlichungstag:
24.10.2001 Patentblatt 2001/43

(51) Int Cl.⁷: $G01N\ 5/04$, G01N 33/38

(21) Anmeldenummer: 00810339.2

(22) Anmeldetag: 19.04.2000

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(71) Anmelder: **Ciments Vigier SA**
**2603 Péry (CH)**

(72) Erfinder: **Jenni, Gilbert**
**3250 Lyss (CH)**

(74) Vertreter: **BOVARD AG - Patentanwälte**
**Optingenstrasse 16**
**3000 Bern 25 (CH)**

(54) **Vorrichtung zur Bestimmung des Wassergehalts von Frischbeton**

(57)    Die Erfindung hat die Aufgabe, eine Vorrichtung zur Verfügung zu stellen, mit der die Bestimmung des Wassergehalts und gegebenenfalls weiterer Kennwerte von Frischbeton sicher, einfach, schnell und zuverlässig erfolgen kann. Die Vorrichtung weist ein Gehäuse (1) zur Aufnahme eines Probegefässes (6) mit einer Probe (7) des Frischbetons und Mittel (5) zum Erhitzen der Probe mittels Mikrowellen auf. Mit einer im Gehäuse (1) eingebauten Waage (4) kann die Gewichtsabnahme der Probe während der Verdampfung des in ihr enthaltenen Wassers gemessen werden. Ein Rechner (22) berechnet den Wassergehalt sowie gegebenenfalls weitere Kennwerte der Probe (7) wie beispielsweise den Wasserzementwert. Die Vorteile der Vorrichtung gegenüber den bekannten Bestimmungsmethoden mit Spiritus- oder Gasbrenner zur Erhitzung der Probe bestehen insbesondere darin, dass die Gewichtsmessungen in einem geschlossenen Gehäuse ablaufen, so dass in dieser Zeit kein Zugriff auf die Probe möglich ist. Die Bestimmung des Wassergehalts kann somit fälschungssicher ablaufen und durch eine automatische Berechnung des Resultats können auch Ablese- und Rechenfehler ausgeschlossen werden.

FIG. 1

Printed by Jouve, 75001 PARIS (FR)

**Beschreibung**

Die Erfindung betrifft eine Vorrichtung zur Bestimmung des Wassergehalts von Frischbeton, enthaltend ein Gehäuse zur Aufnahme eines Probegefässes mit einer Probe des Frischbetons und Mittel zum Erhitzen der Probe mittels Mikrowellen.

Zur Bestimmung des Wassergehalts von Frischbeton wird üblicherweise eine Methode angewendet, bei der eine Probe der Mischung mit einem mit Spiritus oder Gas betriebenen Brenner erwärmt wird, um das in der Probe enthaltene Wasser zu verdampfen. Während des Erwärmens muss die Mischung umgerührt werden. Ein wesentlicher Nachteil dieser Methode ist die von der offenen Flamme ausgehende Gefahr. Die Trocknung findet daher meistens im Freien statt. Aber auch das Mitführen des Brennstoffes zum Ort der Messung ist umständlich und nicht ungefährlich. Um zu zuverlässigen Ergebnissen zu kommen, muss man die Trocknung an einem vor Wind und Niederschlägen geschützten Ort durchführen. Trotzdem ist diese herkömmliche Methode auch in Bezug auf das Messresultat relativ unsicher. Das Messresultat kann absichtlich oder unbeabsichtigt verfälscht werden, beispielsweise durch Hinzufügen oder Entfernen von Material während der Trocknung, durch falsches Ablesen der Waage oder durch Rechenfehler.

Es ist auch bereits bekannt, die Probe mittels Mikrowellen zu erhitzen. Dadurch werden zwar die Sicherheitsprobleme gelöst, nicht aber die Unsicherheiten in bezug auf das Messresultat.

Die Erfindung hat die Aufgabe, die Nachteile des Standes der Technik zu vermeiden und eine Vorrichtung zur Verfügung zu stellen, mit der die Bestimmung des Wassergehalts und gegebenenfalls weiterer Kennwerte von Frischbeton sicher, einfach, schnell und zuverlässig erfolgen kann.

Diese Aufgaben werden dadurch gelöst, dass im Gehäuse Mittel zum Wägen der Probe angeordnet sind, welche mit Rechenmitteln verbunden sind.

Die Vorteile der erfindungsgemässen Vorrichtung gegenüber dem bekannten Stand der Technik bestehen insbesondere darin, dass die Gewichtsmessungen in einem geschlossenen Gehäuse ablaufen, so dass in dieser Zeit kein Zugriff auf die Probe möglich ist. Die Bestimmung des Wassergehalts kann somit fälschungssicher ablaufen und durch eine automatische Berechnung des Resultats können auch Ablese- und Rechenfehler ausgeschlossen werden.

Bevorzugte Ausführungsarten der erfindungsgemässen Vorrichtung sind in den abhängigen Ansprüchen umschrieben.

Die Erfindung wird nachstehend anhand eines Ausführungsbeispiels unter Bezugnahme auf die beiliegenden Zeichnungen näher erläutert. Es zeigt

Figur 1 eine schematische Ansicht einer erfindungsgemässen Vorrichtung und

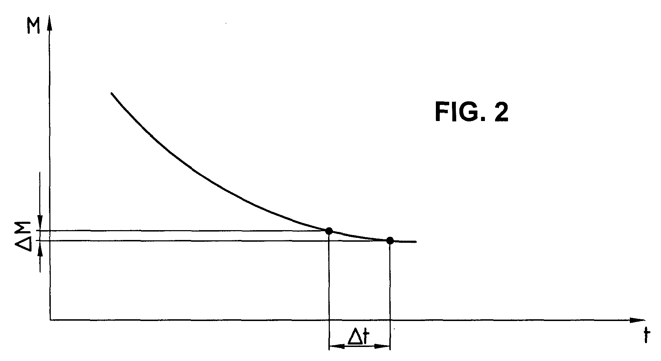

Figur 2 ein Diagramm, das die Abnahme des Gewichts während eines Messvorgangs veranschaulicht.

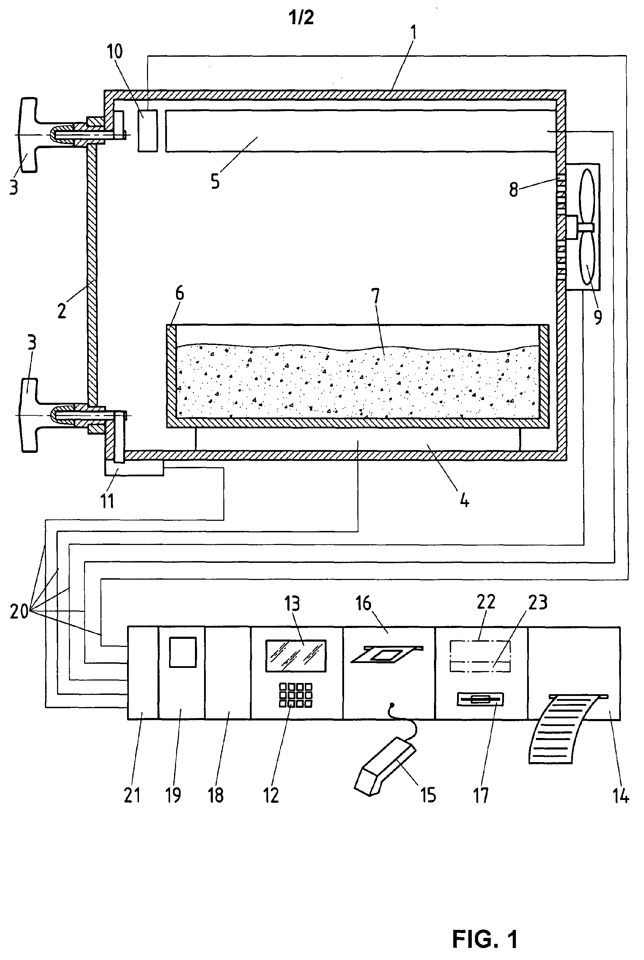

Im oberen Teil der Figur 1 ist in einem schematischen Querschnitt ein Gehäuse 1 zu sehen, das mit einer Türe 2 verschliessbar ist. Eine Schliessvorrichtung 3 ist hier als Drehriegel dargestellt, könnte aber auch beliebig anders gestaltet sein. Die Schliessvorrichtung 3 hat lediglich die Aufgabe, die Türe 2 während des Messvorganges geschlossen zu halten. Ein an der Decke des Gehäuses 1 angebrachter Mikrowellenerzeuger 5 beaufschlagt die Probe 7 mit Mikrowellenenergie, um sie zu erwärmen. Selbstverständlich könnte der Mikrowellenerzeuger auch an einer anderen Stelle des Gehäuses montiert sein, wie dies aus dem Stand der Technik bekannt ist. In der Rückwand des Gehäuses sind Öffnungen 8 angebracht, die es dem bei der Erwärmung der Probe entstehenden Dampf erlauben, aus dem Gehäuse auszutreten. Zum verbesserten Abzug des Dampfes kann zusätzlich ein Ventilator 9 vorgesehen sein. Öffnungen 8 und Ventilator 9 könnten beispielsweise auch in der Decke des Gehäuses 1 angeordnet sein. Ein Schalter 11 wird automatisch betätigt, wenn die Türe 2 geschlossen ist. Die weiter unten beschriebene Steuerung der Vorrichtung ist so gestaltet, dass der Mikrowellenerzeuger 5 nur bei geschlossener Türe 2 betrieben werden kann. Die bisher beschriebenen Komponenten der Vorrichtung können prinzipiell ähnlich aufgebaut und angeordnet sein, wie bei einem Mikrowellengerät, das im Haushalt oder in der Gastronomie zum Erwärmen von Speisen verwendet wird. Selbstverständlich müssen aber die Komponenten für den Einsatz im Bauwesen entsprechend robuster ausgebildet sein.

Auf dem Boden des Gehäuses 1 ruht eine Kraftmesszelle 4, mit der kontinuierlich das Gewicht der Probe 7 des Frischbetons bestimmt werden kann, die in einem Gefäss 6 auf der Messzelle 4 liegt. Die Messzelle muss eine Masse von etwa 40 Kg aufnehmen und deren Gewichtskraft mit einer Genauigkeit von 1‰ bestimmen können. Schliesslich kann im Gehäuse 1 ein Temperaturfühler 10 vorgesehen sein, um gegebenenfalls die Temperatur im Gehäuse zu überwachen und/oder zu steuern.

Die im folgenden beschriebenen Komponenten der Vorrichtung könnten im Gehäuse 1 integriert oder an dieses angebaut sein. Es ist aber vorteilhaft, wenn sie vom Gehäuse 1 getrennt und mit diesem über Leitungen 20 verbunden sind, wie dies in der Figur 1 angedeutet ist. Für die Übermittlung der Mess- und Steuersignale könnte selbstverständlich auch eine drahtlose Verbindung vorgesehen werden. Dadurch wird es beispielsweise möglich, das mit den Proben zu beschickende Gehäuse 1 ausserhalb eines Gebäudes aufzustellen, während die zur Steuerung der Vorrichtung und zur Auswertung der Messung dienenden Komponenten innerhalb des Gebäudes betrieben werden können. Zu diesen Komponenten gehört ein Eingabegerät, beispielsweise eine Tastatur 12, mit der die Messung betreffende Daten eingegeben werden können, wie zum Beispiel die Zementbezeichnung und der Zementgehalt, Art und Menge allfälliger Zuschlagsstoffe, die Auftragsnummer, die Bezeichnung der betreffenden Baustelle und des Objektteils, die Lieferscheinnummer, die Sortennummer und die normierte Bezeichnung des Zements sowie eventuell der Ort der Messung. Ein Rechner 22 ermittelt aus den eingegebenen und den gemessenen Werten die gewünschten Kennzahlen, wie dies weiter unten noch erläutert wird. Der Rechner 22 kann auch zur Steuerung der Vorrichtung eingesetzt werden. Beispielsweise kann er die Erwärmung der probe starten und stoppen. Ein Arbeitsspeicher 23 ist mit dem Rechner verbunden. Eine Anzeigevorrichtung 13, beispielsweise in Form eines mehrzeiligen LCD-Displays oder eines Bildschirms ermöglicht die Darstellung der eingegebenen Daten und der Messresultate. Ferner kann ein Drucker 14 vorgesehen sein, der die eingegebenen Daten und die ermittelten Messresultate ausgibt und diese vorzugsweise automatisch mit Datum und Uhrzeit der Messung versieht. Als zusätzliches Eingabegerät kann ein Barcodeleser 15 vorgesehen sein, mit dem die oben genannten Daten von einem die Probe begleitenden Dokument gelesen werden können. Alternativ oder zusätzlich kann ein Gerät 16 zum Lesen und Beschreiben von Karten, beispielsweise mit einem Chip versehenen Karten vorgesehen sein, für den Fall, dass jede Probe von einer entsprechenden Karte begleitet wird. Ein Diskettenlaufwerk 17 dient dazu, die in der Vorrichtung gespeicherten Daten auf eine Diskette zu übertragen, um sie beispielsweise in einem Computer auszuwerten. Ferner können mit dem Diskettenlaufwerk Daten in die Vorrichtung eingelesen werden, beispielsweise Änderungen der Berechnungsformel. Die Vorrichtung kann auch eine Schnittstelle 18 aufweisen, die deren direkten Anschluss an einen Computer erlaubt. Ein Speisegerät 19 sorgt für die Stromversorgung der Vorrichtung und kann auch einen Akkumulator enthalten, der bei einem Stromausfall oder beim Transport der Vorrichtung dafür sorgt, dass die gespeicherten Daten nicht verloren gehen. Die Leitungen 20 zwischen dem Gehäuse 1 und den anderen Komponenten können mittels eines Steckers 21 mit diesen Komponenten verbunden werden. Schliesslich ist ein mit dem Schalter 11 verbundenes Sicherheitssystem, vorgesehen (nicht dargestellt), das dafür sorgt, dass die Messung erst bei geschlossener Türe beginnt und beim vorzeitigem Öffnen sogleich abbricht.

Die Messungen laufen mit der erfindungsgemässen Vorrichtung wie folgt ab. Zuerst muss die Rohdichte $\rho_0$ des Frischbetons ermittelt werden. Diese Grösse wird später zur Bestimmung des Wassergehalts benötigt. Ein Behälter mit bekanntem Volumen $V_B$ wird leer gewogen und anschliessend mit Frischbeton gefüllt. Nachdem der Frischbeton im Behälter verdichtet wurde, wird dieser noch einmal gewogen. Die Differenz der beiden Wägungen ergibt die Masse $M_B$ des fertig verdichteten Betons. Diese Wägungen können, müssen aber nicht mit der erfindungsgemässen Vorrichtung durchgeführt werden.

$$\rho_0 = \frac{M_B}{V_B}$$

Nun kann mit Hilfe der erfindungsgemässen Vorrichtung der Wassergehalt $W_0$ der Betonmischung ermittelt werden. Ein flacher Behälter 6 wird leer im Gerät gewogen und tariert. In diesem Behälter wird etwa 10 kg Frischbeton eingefüllt und in das Gehäuse 1 gelegt. Nach dem schliessen der Türe werden die notwendigen Angaben wie Auftragsnummer, Baustelle, Objektteil, Lieferscheinnummer, Sortennummer, normierte Bezeichnung des Zements, Ort, Zementart, Zementgehalt und wenn nötig der Gehalt der Zuschlagstoffe durch den Benutzer eingegeben. Nun wird automatisch die Betonmenge gewogen und danach beginnt unter ständiger Gewichtsmessung das Trocknen des Betons. Der Trocknungsvorgang ist abgeschlossen, wenn die Abnahme des Gewichts pro Zeiteinheit einen vorgegebenen minimalen Wert unterschreitet, wie dies in Figur dargestellt ist. Danach erfolgt die automatische Berechnung des Wassergehalts $W_0$ der Probe nach der Formel

$$W_0 = \frac{M_0 - M_1}{M_0} \cdot \rho_0$$

wobei $M_0$ die Masse der Frischbetonprobe ist, $M_1$ die Masse der getrockneten Probe und $\rho_0$ die zuvor ermittelte Rohdichte des verdichteten Betons. Sofern der Zementwert Z bekannt ist und in die Vorrichtung eingegeben wird, kann diese auch automatisch den Wasserzementwert WZ ermitteln nach der Formel

$$WZ_0 = \frac{W_0}{Z}$$

Die gemessenen und berechneten Werte werden je nach Wunsch in der Vorrichtung gespeichert, auf der Anzeigevorrichtung 13 angezeigt und/oder vom Drucker 14 ausgedruckt.

**Patentansprüche**

1. Vorrichtung zur Bestimmung des Wassergehalts von Frischbeton, enthaltend ein Gehäuse (1) zur Aufnahme eines Probegefässes (6) mit einer Probe (7) des Frischbetons und Mittel (5) zum Erhitzen der Probe mittels Mikrowellen, **dadurch gekennzeichnet, dass** im Gehäuse (1) Mittel (4) zum Wägen der Probe (7) angeordnet sind, welche mit Rechenmitteln (22) verbunden sind.

2. Vorrichtung nach Anspruch 1, **dadurch gekennzeichnet, dass** sie Eingabemittel (12, 15, 16, 17) zum Eingeben von Daten und Steuerbefehlen aufweist.

3. Vorrichtung nach Anspruch 2, **dadurch gekennzeichnet, dass** die Eingabemittel eine Tastatur (12) aufweisen.

4. Vorrichtung nach Anspruch 2, **dadurch gekennzeichnet, dass** die Eingabemittel ein Lesegerät (15, 16) aufweisen.

5. Vorrichtung nach Anspruch 4, **dadurch gekennzeichnet, dass** das Lesegerät (15) zum Erkennen von optischen Zeichen ausgebildet ist.

6. Vorrichtung nach Anspruch 4, **dadurch gekennzeichnet, dass** das Lesegerät (16) zum Lesen von Magnetkarten oder Chipkarten ausgebildet ist.

7. Vorrichtung nach Anspruch 2, **dadurch gekennzeichnet, dass** die Eingabemittel ein Diskettenlaufwerk (17) aufweisen.

8. Vorrichtung nach einem der vorangehenden Ansprüche, **dadurch gekennzeichnet, dass** sie Speichermittel (17, 23) zum Speichern der eingegebenen und berechneten Daten aufweist.

9. Vorrichtung nach einem der vorangehenden Ansprüche, **dadurch gekennzeichnet, dass** sie Ausgabemittel (13, 14, 16, 17) zum Ausgeben der eingegebenen und berechneten Daten aufweist.

10. Vorrichtung nach Anspruch 9, **dadurch gekennzeichnet, dass** die Ausgabemittel eine Anzeigevorrichtung (17) und/oder einen Drucker (14) enthalten.

11. Vorrichtung nach Anspruch 9, **dadurch gekennzeichnet, dass** die Ausgabemittel ein Gerät (16) Beschreiben von Magnetkarten oder Chipkarten aufweisen.

12. Vorrichtung nach Anspruch 8, **dadurch gekennzeichnet, dass** die Ausgabemittel ein Diskettenlaufwerk (17) aufweisen.

13. Vorrichtung nach einem der Ansprüche 1 bis 12, **dadurch gekennzeichnet, dass** die Eingabemittel (12, 15, 16, 17) und/oder die Speichermittel (17, 23) und/oder die Ausgabemittel (13, 14, 16) vom Gehäuse (1) getrennt und mit diesem über Verbindungsmittel, beispielsweise ein Kabel (20) verbunden sind.

1/2

FIG. 1

FIG. 2

**Europäisches Patentamt**

## EUROPÄISCHER RECHERCHENBERICHT

Nummer der Anmeldung

EP 00 81 0339

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 5 983 711 A (PAPPAS WILLIAM D ET AL) 16. November 1999 (1999-11-16) * Spalte 1, Zeile 6 - Zeile 11 * * Spalte 2, Zeile 35 - Zeile 46 * * Spalte 2, Zeile 56 - Spalte 3, Zeile 8 * * Spalte 3, Zeile 55 - Spalte 4, Zeile 6 * * Spalte 4, Zeile 17 - Zeile 25 * * Spalte 5, Zeile 43 - Zeile 61 * --- | 1-13 | G01N5/04 G01N33/38 |
| X | US 4 750 143 A (HEITZ GEORGES ET AL) 7. Juni 1988 (1988-06-07) * Spalte 1, Zeile 48 - Spalte 2, Zeile 29 * * Spalte 5, Zeile 15 - Zeile 32 * --- | 1-13 | |
| A | US 5 724 257 A (GEROSA HORACIO MARCELO ET AL) 3. März 1998 (1998-03-03) * Abbildung 2 * ----- | 1-13 | |

RECHERCHIERTE SACHGEBIETE (Int.Cl.7)

G01N

Der vorliegende Recherchenbericht wurde für alle Patentansprüche erstellt

| Recherchenort | Abschlußdatum der Recherche | Prüfer |
|---|---|---|
| BERLIN | 14. September 2000 | Navas Montero, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 00 81 0339

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-09-2000

| Im Recherchenbericht angeführtes Patentdokument | Datum der Veröffentlichung | Mitglied(er) der Patentfamilie | Datum der Veröffentlichung |
|---|---|---|---|
| US 5983711    A | 16-11-1999 | KEINE | |
| US 4750143    A | 07-06-1988 | FR    2573530 A<br>AT      51079 T<br>AU     581944 B<br>AU    4998185 A<br>BR    8505850 A<br>CA    1251278 A<br>DE    3576565 D<br>EP    0186604 A<br>ES     549086 D<br>ES    8609710 A<br>FR    2642167 A<br>JP   61130854 A<br>ZA    8508884 A | 23-05-1986<br>15-03-1990<br>09-03-1989<br>29-05-1986<br>12-08-1986<br>14-03-1989<br>19-04-1990<br>02-07-1986<br>16-07-1986<br>16-12-1986<br>27-07-1990<br>18-06-1986<br>30-07-1986 |
| US 5724257    A | 03-03-1998 | DE   19781585 T<br>JP 2000509491 T<br>WO    9740359 A | 12-05-1999<br>25-07-2000<br>30-10-1997 |

EPO FORM P0461